# EUROPEAN PATENT SPECIFICATION

(11) **EP 2 292 225 B1**
(45) Date of publication and mention of the grant of the patent: **09.05.2012**
(21) Application number: 10175858.9
(22) Date of filing: 31.03.1998
(51) Int. Cl.: A61K 31/335, A61L 31/16, A61P 7/00, A61F 2/06, A61K 31/16

(54) **Dosage form comprising taxol in crystalline form**
Darreichungsform enthaltend Taxol in kristalliner Form
Forme de dosage comprénant du taxol en forme cristalline

(30) Priority: 31.03.1997 US 829685; 31.03.1997 US 827991
(43) Date of publication of application: 09.03.2011
(62) Divisional of application: 09004768.9
(73) Proprietor: BOSTON SCIENTIFIC SCIMED LIMITED, St. Michael (BB)
(72) Inventor: Kunz, Lawrence, L., Ellensburg, WA 98926 (US); Klein, Richard, A., Edmonds, WA 98026 (US); Reno, John, M., Brier, WA 98036 (US)
(74) Representative: Peterreins, Frank

(56) References cited:
- WO-A-92/11890
- WO-A-94/16706
- WO-A-96/25176
- KUNZ, L.L. ET AL: "Efficacy of cytochalasin B in inhibiting coronary restenosis caused by chronic remodelling after balloon trauma in swine", JOURNAL OF THE AMERICAN COLLEGE OF CARDIOOGY, vol. 0, 1995, page 302A, XP002075725,
- CAMENZIND, E. ET AL: "Use of locally delivered conventional drug therapies", SEMIN. INTERVENT. CARDIOL., vol. 1, no. 1, March 1996 (1996-03), pages 67-76, XP002075726,
- LEVY, R.J. ET AL: "Strategies for treating arterial restenosis using polymeric controlled release implants", 1994, BIOTECHNOLOGY AND BIOACTIVE POLYMERS; PLENUM PRESS, NEW YORK; EDITORS GEBELEIN, C. AND CARRAHER, C., XP002076528, * page 259 - page 268 * * the whole document *

## Description

### Background of the Invention

Percutaneous transluminal coronary angioplasty (PTCA) is widely used as the primary treatment modality in many patients with coronary artery disease. PTCA can relieve myocardial ischemia in patients with coronary artery disease by reducing lumen obstruction and improving coronary flow. The use of this surgical procedure has grown rapidly, with 39,000 procedures performed in 1983, nearly 150,000 in 1987, 200,000 in 1988, 250,000 in 1989, and over 500,000 PTCAs per year are estimated by 1994 (Popma et al., Amer. J. Med., 88: 16N-24N (1990); Fanelli et al, Amer. Heart Jour, 119: 357-368 (1990); Johnson et al., Circulation, 78 (Suppl. II): II-82 (1988)). Stenosis following PTCA remains a significant problem, with from 25% to 35% of the patients developing restenosis within 1 to 3 months. Restenosis results in significant morbidity and mortality and frequently necessitates further interventions such as repeat angioplasty or coronary bypass surgery. As of 1993, no surgical intervention or post-surgical treatment has proven effective in preventing restenosis.

The processes responsible for stenosis after PTCA are not completely understood but may result from a complex interplay among several different biologic agents and pathways. Viewed in histological sections, restenotic lesions may have an overgrowth of smooth muscle cells in the intimal layers of the vessel (Johnson et al., Circulation, 78 (Suppl. II): II-82 (1988)). Several possible mechanisms for smooth muscle cell proliferation after PTCA have been suggested (Popma et al., Amer. J. Med., 88: 16N-24N (1990); Fanelli et al, Amer, Heart Jour., 119: 357-368 (1990); Liu et al., Circulation, 79: 1374-1387 (1989); Clowes et al., Circ. Res., 56: 139-145 (1985)).

Compounds that reportedly suppress smooth muscle proliferation in *vitro* (Liu et al., Circulation, 79: 1374-1387 (1989); Goldman et al., Atherosclerosis, 65: 215-225 (1987); Wolinsky et al., JACC, 15 (2): 475-481 (1990)) may have undesirable pharmacological side effects when used *in vivo.* Heparin is an example of one such compound, which reportedly inhibits smooth muscle cell proliferation *in vitro* but when used *in vivo* has the potential adverse side effect of inhibiting coagulation. Heparin peptides, while having reduced anti-coagulant activity, have the undesirable pharmacological property of having a short pharmacological half-life. Attempts have been made to solve such problems by using a double balloon catheter, i.e., for regional delivery of the therapeutic agent at the angioplasty site (e.g., Nabel et al., Science, 244: 1342-1344 (1989); U.S. Patent No. 4,824,436), and by using biodegradable materials impregnated with a drug, i.e., to compensate for problems of short half-life (e.g., Middlebrook et al., Biochem. Pharm., 38 (18): 3101-3110 (1989); U.S. Patent No. 4,929,602).

At least five considerations would, on their face, appear to preclude use of inhibitory drugs to prevent stenosis resulting from overgrowth of smooth muscle cells. First, inhibitory agents may have systemic toxicity that could create an unacceptable level of risk for patients with cardiovascular disease. Second, inhibitory agents might interfere with vascular wound healing following surgery and that could either delay healing or weaken the structure or elasticity of the newly healed vessel wall. Third, inhibitory agents which kill smooth muscle cells could damage surrounding endothelium and/or other medial smooth muscle cells. Dead and dying cells also release mitogenic agents that might stimulate additional smooth muscle cell proliferation and exacerbate stenosis. Fourth, delivery of therapeutically effective levels of an inhibitory agent may be problematic from several standpoints: namely, a) delivery of a large number of molecules into the intercellular spaces between smooth muscle cells may be necessary, i.e., to establish favorable conditions for allowing a therapeutically effective dose of molecules to cross the cell membrane; b) directing an inhibitory drug into the proper intracellular compartment, i.e., where its action is exerted, may be difficult to control; and, c) optimizing the association of the inhibitory drug with its intracellular target, e.g, a ribosome, while minimizing intercellular redistribution of the drug, e.g. to neighboring cells, may be difficult. Fifth, because smooth muscle cell proliferation takes place over several weeks it would appear *a priori* that the inhibitory drugs should also be administered over several weeks, perhaps continuously, to produce a beneficial effect.

As is apparent from the foregoing, many problems remain to be solved in the use of inhibitory drugs to effectively treat smooth muscle cell proliferation. Thus, there is a need for a method to inhibit or reduce stenosis due to proliferation of vascular smooth muscle cells following traumatic injury to vessels such as occurs during vascular surgery. There is also a need to deliver compounds to vascular smooth muscle cells which exert inhibitory effects over extended periods of time.

WO 92/11890 A1 discloses balloon catheters which include a coating of body affecting chemicals on the exterior of the balloon.

WO 94/16706 A1 and WO 96/25176 A1 disclose methods for inhibiting stenosis comprising administering a therapeutic agent that inhibits a cellular activity to the host. Taxol is mentioned as a possible therapeutic agent.

### Summary of the Invention

The present invention relates to crystalline taxol having a crystal size of 0.1 micron to 1 mm for use in inhibiting or reducing diminution in vessel lumen volume in a procedurally traumatized mammalian blood vessel, in particular wherein taxol is administered *in situ* by means of an implantable device wherein taxol is releasably embedded in, coated on or embedded in and coated on the implantable device. The present invention also relates to a dosage form comprising a taxol for use in inhibiting or reducing diminution in vessel lumen area of a mammalian blood vessel, wherein the Taxol is in substantially crystalline form and wherein the crystal size of the taxol is 0.1 micron to 1 mm.

Said dosage form may be used in a method for inhibiting or reducing diminution in vessel lumen volume in a procedurally traumatized mammalian blood vessel. The method comprises administering to the blood vessel of a mammal an effective amount of taxol, wherein the taxol is in substantially pure substantially crystalline form and wherein the crystals are of a size which results in sustained release of taxol . Preferably, the crystals are of a size of about 0.1 micron to about 10 mm, preferably about 1 micron to about 25 micron, in size. Methods to determine the size of crystals useful for sustained release are well known to the art. Preferably, taxol is administered *in situ,* by means of an implantable device, wherein the cytoskeletal inhibitor is releasably embedded in, coated on, or embedded in and coated on, the implantable device. Preferably, the crystalline cytoskeletal inhibitor is releasably embedded in, or dispersed in, a adventitial wrap, e.g., a silicone membrane. A preferred therapeutic implantable device of the invention comprises about 1 to about 70, preferably about 2 to about 50, and more preferably about 3 to about 10, weight percent of taxol per weight percent of the adventitial wrap. Alternatively, a preferred therapeutic implantable device of the invention comprises about 30 to about 70, preferably about 30 to about 60, and more preferably about 30 to about 50, weight percent of taxol per weight percent of the adventitial wrap. Alternatively, taxol may be suspended in a vehicle which yields a solution comprising the crystals, i.e., it is a saturated solution.

The invention also provides therapeutic devices comprising the dosage form of the invention in an amount effective to inhibit stenosis or reduce restenosis .

### Brief Description of the Drawings

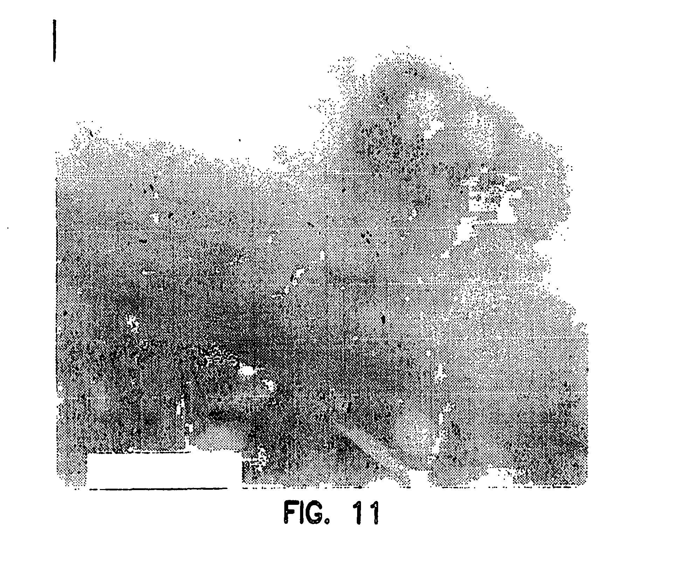

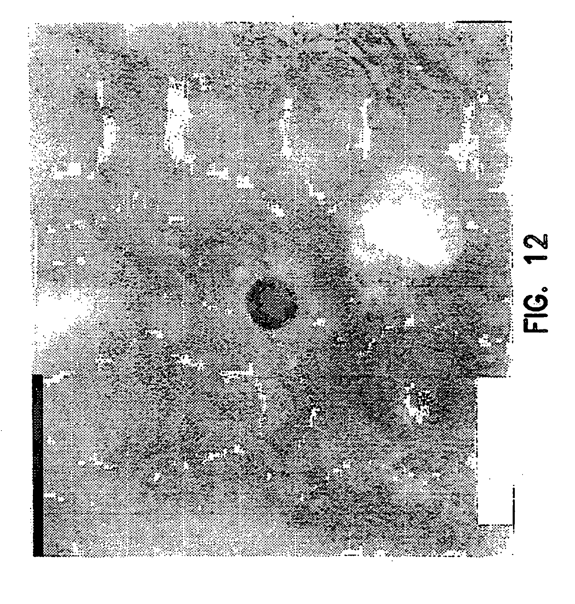

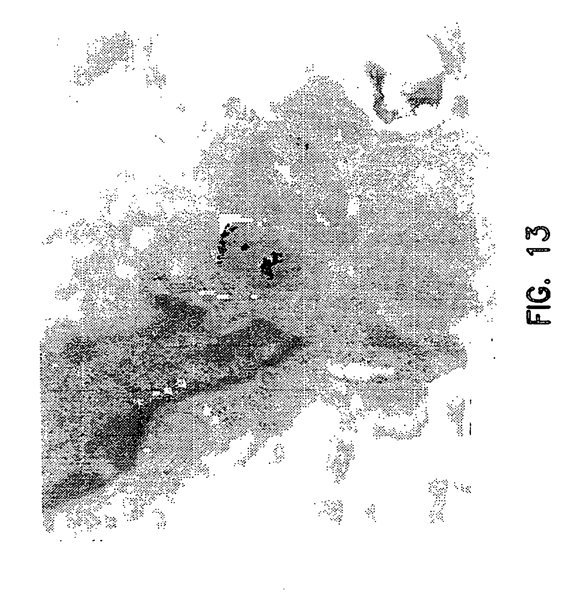

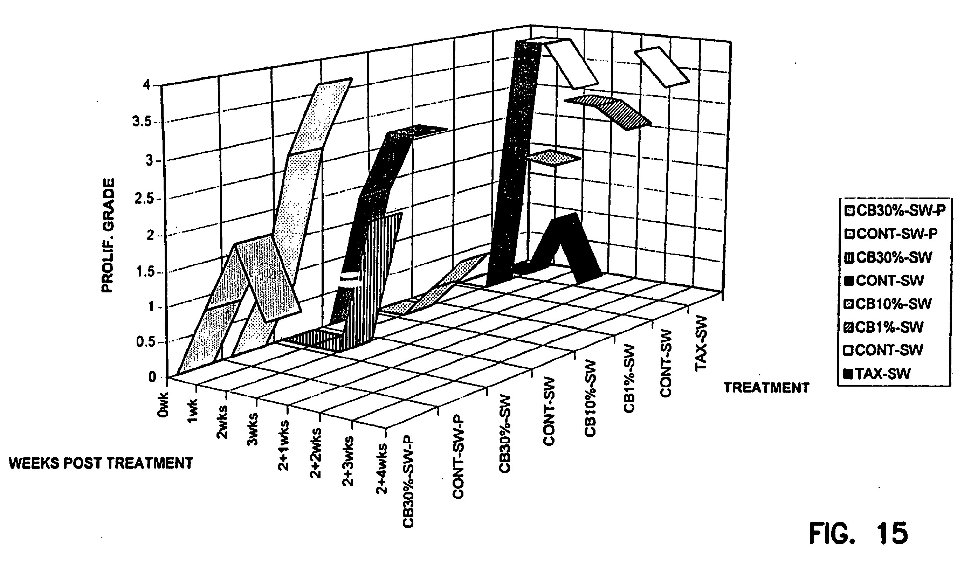

FIGURE 15 is a graph depicting the inhibition of smooth muscle cell proliferation in traumatized vessels over time by cytochalasin B (CB) or taxol (TAX) administered in silicone wraps (SW).

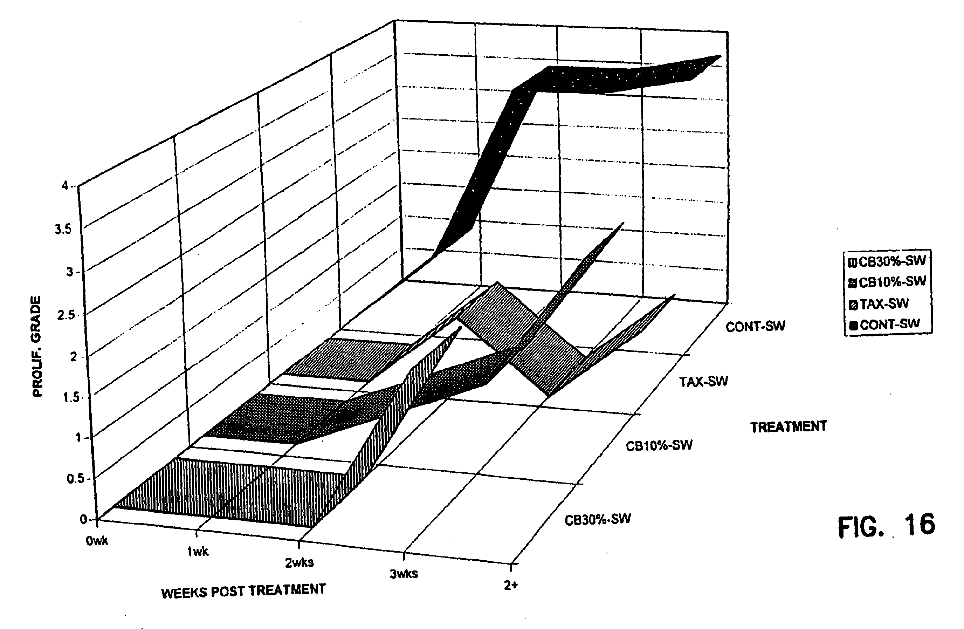

FIGURE 16 is a graph depicting the inhibition of smooth muscle cell proliferation in traumatized vessels over time by 10% or 30% wt/wt CB in SW or 5% wt/wt TAX in SW.

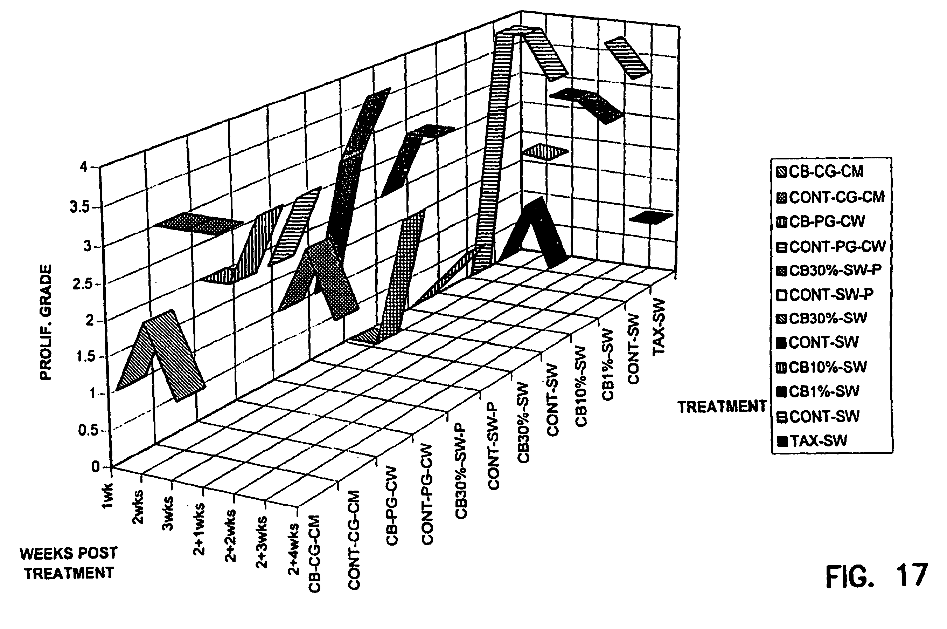

FIGURE 17 is a graph depicting the inhibition of smooth muscle cell proliferation in traumatized vessels over time by CB or TAX in silicone, CB in a collagen gel supported by a bovine collagen mesh (CG-CM) or CB in a pluronic gel supported by a bovine collagen mesh (PG-CW).

### Detailed Description of the Invention

### Definitions

"Therapeutic agent" includes any moiety capable of exerting a therapeutic or prophylactic effect in the present method.

As used herein, "substantially" pure means at least about 90%, preferably at least about 98%, and more preferably at least about 99%, free of contaminants when assayed by methods conventionally employed by the art.

As used herein, "substantially" solid or crystalline means at least about 90%, preferably at least about 98%, and more preferably at least about 99%, free of non-solid or non-crystalline forms or phases when assayed by methods conventionally employed by the art.

"Migration" of smooth muscle cells means movement of these cells *in vivo* from the medial layers of a vessel into the intima, which may also be studied *in vitro* by following the motion of a cell from one location to another (e.g., using time-lapse cinematography or a video recorder and manual counting of smooth muscle cell migration out of a defined area in the tissue culture over time).

"Proliferation" means an increase in cell number, i.e., by mitosis of the cells. As used herein "smooth muscle cells" does not refer to neoplastic vascular smooth muscle cells, i.e., cancer cells.

"Implantable device" means any material that is capable of retaining and releasing a therapeutic agent so as to deliver it *in situ* in a controlled fashion to a mammalian vessel. An implantable device includes devices which are placed in the lumen of the vessel, e.g., an indwelling catheter or stent, or on the exterior of a vessel, e.g., an adventitial wrap, mesh or covering, or which become a part of the vessel itself, for example to replace a portion of a diseased or traumatized vessel, e.g., a synthetic graft. The implantable device may comprise the therapeutic agent in a form which is releasably embedded in and/or coated on the device. The therapeutic agent may also be releasably embedded in and/or coated on a pharmaceutically acceptable release carrier matrix, which may be applied to and/or embedded in the device or administered directly to a vessel. For example, a matrix useful in the practice of the invention includes, but is not limited to, microparticles, nanoparticles, a gel, a paste, or a permeable membrane. An implantable device may be implanted for a limited amount of time, e.g., catheter or infusion needle delivery of a therapeutic agent, or for a prolonged period of time, e.g., a stent or graft. Vessels, into which the implantable device of the invention may be inserted, include, but are not limited to, coronary, femoral, carotid and peripheral vessels.

"Sustained release" means a dosage form designed to release a therapeutic agent therefrom for a time period from about 0.0005 to about 180, preferably from about 1-3 to about 150, and more preferably from about 30 to about 120, days. Release over a longer time period is also contemplated as "sustained release" in the context of the present invention. Moreover, it is contemplated that the invention can be practiced with a locally or systemically administered sustained release dosage form.

"Dosage form" includes a formulation comprising a free (non-targeted or non-binding partner associated) therapeutic agent, as well as a sustained release formulation comprising a therapeutic agent. For example, sustained release formulations can comprise microparticles or nanoparticles, microemulsions, biodegradable or non-biodegradable polymeric materials, or any combination thereof, comprising a therapeutic agent dispersed therein, as well as crystalline forms of the therapeutic agent. A targeted or binding partner associated dosage form of the invention includes a sustained release therapeutic formulation comprising microparticles or nanoparticles, microemulsions, and/or biodegradable or non-biodegradable polymeric materials. The sustained release dosage form is linked to one or more binding proteins or peptides, so as to deliver a therapeutic agent dispersed therein to a target cell population which binds to the binding protein or peptide.

As referred to herein, "taxol" includes taxol as well as functional analogs, equivalents or derivatives thereof. For example, derivatives and analogs of taxol include, but are not limited to, taxotere, baccatin, 10-deacetyltaxol, 7-xylosyl-10-deacetyltaxol, cephalomannine, 10-deacetyl-7-epitaxol, 7 epitaxol, 10-deacetylbaccatin III, 10-deacetylcephaolmannine and analogs or derivatives disclosed in Kingston et al. (New Trends in Nat Prod. Chem., 26, 219 (1986)), Bringli et al. (WO 93/17121), Golik et al. (EPA 639577), Kelly et al. (WO 95/20582), and Cassady and Dourous (eds., In: Anticancer Agents Based on Natural Product Models, Academic Press, NY (1980)), Methods for preparing taxol and numerous analogs and derivatives thereof are well known to the art.

As referred to herein, smooth muscle cells and pericytes include those cells derived from the medial layers of vessels and adventitial vessels which proliferate in intimal hyperplastic vascular sites following injury, such as that caused during PTCA. Characteristics of smooth muscle cells include a histological morphology (under light microscopic examination) of a spindle shape with an oblong nucleus located centrally in the cell with nucleoli present and myofibrils in the sarcoplasm. Under electron microscopic examination, smooth muscle cells have long slender mitochondria in the juxtanuclear sarcoplasm, a few tubular elements of granular endoplasmic reticulum, and numerous clusters of free ribosomes. A small Golgi complex may also be located near one pole of the nucleus. The majority of the sarcoplasm is occupied by thin, parallel myofilaments that may be, for the most part, oriented to the long axis of the muscle cell. These actin containing myofibrils may be arranged in bundles with mitochondria interspersed among them. Scattered through the contractile substance of the cell may also be oval dense areas, with similar dense areas distributed at intervals along the inner aspects of the plasmalemma.

Characteristics of pericytes include a histological morphology (under light microscopic examination) characterized by an irregular cell shape. Pericytes are found within the basement membrane that surrounds vascular endothelial cells and their identity may be confirmed by positive immuno-staining with antibodies specific for alpha smooth muscle actin.(e.g., anti-alpha-sm1, Biomakor, Rehovot, Israel), HMW-MAA, and pericyte ganglioside antigens e.g., MAb 3G5 (Schlingemann et al., Am. J._Pathol., 136: 1393-1405 (1990)); and, negative immuno-staining with antibodies to cytokeratins (i.e., epithelial and fibroblast markers) and von Willdebrand factor (i.e., an endothelial marker). Both vascular smooth muscle cells and pericytes are positive by immunostaining with the NR-AN-01 monoclonal antibody.

As used herein, the term "procedural vascular trauma" includes the effects of surgical/mechanical interventions into mammalian vasculature, but does not include vascular trauma due to the organic vascular pathologies, i.e., diseases and infections.

Thus, procedural vascular traumas within the scope of the present treatment method include (1) organ transplantation, such as heart, kidney, liver and the like, e.g., involving vessel anastomosis; (2) vascular surgery, e.g., coronary bypass surgery, biopsy, heart valve replacement, atheroectomy, thrombectomy, and the like; (3) transcatheter vascular therapies (TVT) including angioplasty, e.g., laser angioplasty and PTCA procedures, employing balloon catheters, and indwelling catheters; (4) vascular grafting using natural or synthetic materials, such as in saphenous vein coronary bypass grafts, dacron and venous grafts used for peripheral arterial reconstruction, etc.; (5) placement of a mechanical shunt, e.g., a PTFE hemodialysis shunt used for arteriovenous communications; and (6) placement of an intravascular stent, which may be metallic, plastic or a biodegradable polymer. See U.S. patent application Serial No. 08/389,712, filed February 15, 1995, which is incorporated by reference herein. For a general discussion of implantable devices and biomaterials from which they can be formed, see H. Kambic et al., "Biomaterials in Artificial Organs", Chem Eng. News, 30 (April 14,1986), the disclosure of which is incorporated by reference herein.

### Sustained Released Dosage Forms

Sustained release dosage forms of the invention may comprise microparticles, nanoparticles or microemulsions having a therapeutic agent dispersed therein or may comprise the therapeutic agent in pure, preferably crystalline, solid form. For sustained release administration, microparticle dosage forms comprising pure, preferably crystalline, therapeutic agents are preferred. The therapeutic dosage forms of this aspect of the present invention may be of any configuration suitable for sustained release. Preferred sustained release therapeutic dosage forms exhibit one or more of the following characteristics:
- microparticles (e.g., from about 0.01 micrometers to about 200 micrometers in diameter, preferably from about 0.5 to about 50 micrometers, and more preferably from about 2 to about 15 micrometers) or nanoparticles (e.g., from about 0.01 nanometer to about 1000 nanometers in diameter, preferably from about 50 to about 200 nanometers), free flowing powder structure;
- biodegradable structure designed to biodegrade over a period of time preferably between from about 0.5 to about 180 days, preferably from about 1-3 to about 150 days, or non-biodegradable structure to allow therapeutic agent diffusion to occur over a time period of between from about 0.5 to about 180 days, more preferably from about 30 to about 120 days;
- biocompatible with target tissue and the local physiological environment into which the dosage form to be administered, including yielding biocompatible biodegradation products;
- facilitate a stable and reproducible dispersion of therapeutic agent therein, preferably to form a therapeutic agent-polymer matrix, with active therapeutic agent release occurring by one or both of the following routes: (1) diffusion of the therapeutic agent through the dosage form (when the therapeutic agent is soluble in the shaped polymer or polymer mixture defining the dimensions of the dosage form); or (2) release of the therapeutic agent as the dosage form biodegrades; and/or
- for targeted dosage forms, having, preferably, from about 1 to about 10,000 binding protein/peptide to dosage form bonds and more preferably, a maximum of about 1 binding peptide to dosage form bond per 150 square angstroms of particle surface area. The total number of binding protein/peptide to dosage form bonds depends upon the particle size used. The binding proteins or peptides are capable of coupling to the particles of the therapeutic dosage form through covalent ligand sandwich or non-covalent modalities as set forth herein.

Nanoparticle sustained release therapeutic dosage forms are preferably biodegradable and, optionally, bind to the vascular smooth muscle cells and enter those cells, primarily by endocytosis. The biodegradation of the nanoparticles occurs over time (e.g., 30 to 120 days) in prelysosomic vesicles and lysosomes. Preferred larger microparticle therapeutic dosage forms of the present invention release the therapeutic agents for subsequent target cell uptake with only a few of the smaller microparticles entering the cell by phagocytosis. A practitioner in the art will appreciate that the precise mechanism by which a target cell assimilates and metabolizes a dosage form of the present invention depends on the morphology, physiology and metabolic processes of those cells. The size of the particle sustained release therapeutic dosage forms is also important with respect to the mode of cellular assimilation. For example, the smaller nanoparticles can flow with the interstitial fluid between cells and penetrate the infused tissue. The larger microparticles tend to be more easily trapped interstitially in the infused primary tissue, and thus are useful to deliver anti-proliferative therapeutic agents.

Preferred sustained release dosage forms of the present invention comprise biodegradable microparticles or nanoparticles. More preferably, biodegradable microparticles or nanoparticles are formed of a polymer containing matrix that biodegrades by random, nonenzymatic, hydrolytic scissioning to release therapeutic agent, thereby forming pores within the particulate structure.

Polymers derived from the condensation of alpha hydroxycarboxylic acids and related lactones are preferred for use in the present invention. A particularly preferred moiety is formed of a mixture of thermoplastic polyesters (e.g., polylactide or polyglycolide) or a copolymer of lactide and glycolide components, such as poly(lactide-co-glycolide). An exemplary structure, a random poly(DL-lactide-co-glycolide), is shown below, with the values of x and y being manipulable by a practitioner in the art to achieve desirable microparticle or nanoparticle properties.

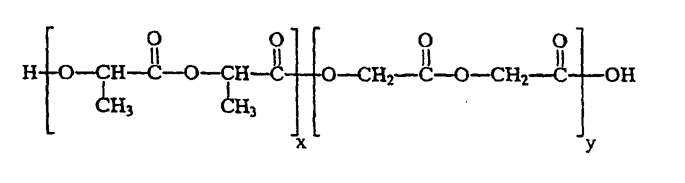

Other agents suitable for forming particulate dosage forms of the present invention include polyorthoesters and polyacetals (polymer Letters, 18:293 (1980) and polyorthocarbonates (U.S. Patent No. 4,093,709) and the like.

Preferred lactic acid/glycolic acid polymer containing matrix particles of the present invention are prepared by emulsion-based processes, that constitute modified solvent extraction processes, see, for example, processes described by Cowsar et al., "Poly(Lactide-Co-Glycolide) Microcapsules for Controlled Release of Steroids," Methods Enzymology, 112:101-116, 1985 (steroid entrapment in microparticles); Eldridge et al., "Biodegradable and Biocompatible Poly(DL-Lactide-Co-Glycolide) Microspheres as an Adjuvant for Staphylococcal Enterotoxin B Toxoid Which Enhances the Level of Toxin-Neutralizing Antibodies," Infection and Immunity, 59:2978-2986, 1991 (toxoid entrapment); Cohen et al., "Controlled Delivery Systems for Proteins Based on Poly(Lactic/Glycolic Acid) Microspheres," Pharmaceutical Research, 8(6):713-720, 1991 (enzyme entrapment); and Sanders et al., "Controlled Release of a Luteinizing Hormone-Releasing Hormone Analogue from Poly(D,L-Lactide-Co-Glycolide) Microspheres," J. Pharmaceutical Science, 73(9):1294-1297,1984 (peptide entrapment).

In general, the procedure for forming particle dosage forms of the present invention involves dissolving the polymer in a halogenated hydrocarbon solvent, dispersing a therapeutic agent solution (preferably aqueous)therein, and adding an additional agent that acts as a solvent for the halogenated hydrocarbon solvent but not for the polymer. The polymer precipitates out from the polymer-halogenated hydrocarbon solution onto droplets of the therapeutic agent containing solution and entraps the therapeutic agent. Preferably the therapeutic agent is substantially uniformly dispersed within the sustained release dosage form of the present invention. Following particle formation, they are washed and hardened with an organic solvent. Water washing and aqueous nonionic surfactant washing steps follow, prior to drying at room temperature under vacuum.

For biocompatibility purposes, particulate dosage forms, characterized by a therapeutic agent dispersed in the matrix of the particles, are sterilized prior to packaging, storage or administration. Sterilization may be conducted in any convenient manner therefor. For example, the particles can be irradiated with gamma radiation, provided that exposure to such radiation does not adversely impact the structure or function of the therapeutic agent dispersed in the therapeutic agent-polymer matrix or the binding protein/peptide attached thereto. If the therapeutic agent or binding protein/peptide is so adversely impacted, the particle dosage forms can be produced under sterile conditions.

Release of the therapeutic agent from the particle dosage forms of the present invention can occur as a result of both diffusion and particle matrix erosion. The biodegradation rate directly effects the kinetics of therapeutic agent release. The biodegradation rate is regulable by alteration of the composition or structure of the sustained release dosage form. For example, alteration of the lactide/glycolide ratio in preferred dosage forms of the present invention can be conducted, as described by Tice et al., "Biodegradable Controlled-Release Parenteral Systems," Pharmareutical Technology, pp. 26-35, 1984; by inclusion of agents that alter the rate of polymer hydrolysis, such as citric acid and sodium carbonate, as described by Kent et al., "Microencapsulation of Water Soluble Active Polypeptides," U.S. Patent No. 4,675,189; by altering the loading of therapeutic agent in the lactide/glycolide polymer, the degradation rate being inversely proportional to the amount of therapeutic agent contained therein, by judicious selection of an appropriate analog of a common family of therapeutic agents that exhibit different potencies so as to alter said core loadings; and by variation of particle size, as described by Beck et al., "Poly(DL-Lactide-Co-Glycolide)/Norethisterone Microcapsules: An Injectable Biodegradable Contraceptive," Biol. Reprod., 28:186-195, 1983, or the like. All of the aforementioned methods of regulating biodegradation rate influence the intrinsic viscosity of the polymer containing matrix, thereby altering the hydration rate thereof.

The preferred lactide/glycolide structure is biocompatible with the mammalian physiological environment. Also, these preferred sustained release dosage forms have the advantage that biodegradation thereof forms lactic acid and glycolic acid, both normal metabolic products of mammals.

To prepare one embodiment of the invention, taxol is incorporated into biodegradable poly (DL-lactide-co-glycolide) microparticles or into nanoparticles. The microparticles are about 1 to about 50 µm, preferably 4 µm to about 15 µm, and more preferably about 2 to about 15 µm, in diameter. The nanoparticles are about 5 to about 500 nanometers, preferably about 10 to about 250 nanometers, and more preferably about 50 to about 200 nanometers, in diameter. The microparticles or nanoparticles comprising taxol can be further embedded in or on an implantable device, e.g., in a stent coating, or delivered in a suitable liquid vehicle by an implantable device, e.g., via an infusion catheter. Preferably, the sustained release dosage form is biodegradable and, preferably, biodegrades over about 30-120 days. The sustained release dosage form is preferably administered during the procedural vascular trauma.

A preferred sustained release dosage form of the invention comprises biodegradable microparticles, preferably about 2 to about 15 µm in diameter, which are tissue compatible and physically compatible with an implantable device, e.g., a needle infusion catheter or a microinfusion catheter. Another preferred sustained release dosage form of the invention comprises biodegradable nanoparticles, preferably about 50 to about 200 nanometers in diameter, which are tissue compatible and physically compatible with an implantable device, e.g., a needle infusion catheter or a microinfusion catheter. To deliver the sustained release dosage forms by catheter, catheter pore or hole sizes are preferably about 0.1 to about 8 µm, more preferably about 0.2 to about 0.8 µm, in diameter.

The cellular concentration of the cytoskeletal inhibitor that is attained in the tunica media and/or intima of the treated vessel is effective to inhibit vascular smooth muscle cell proliferation and migration. The inhibition of the smooth muscle cells results in a more rapid and complete re-endothelialization after a procedural vascular trauma, e.g., intraventional placement of the stent. The increased rate of re-endothelialization reduces loss in luminal cross-sectional area or diameter and reduces decreases in blood flow.

The sustained release dosage form of the invention comprises a pure, solid crystalline form of taxol.

The sustained release dosage form of the present invention preferably further comprises a tissue-compatible pharmaceutically acceptable matrix carrier that provides a supporting structure for the crystals, e.g., a shaped body of silicone, collagen gel retrained in a collagen mesh, pluronic gel retained in a collagen mesh, or mannitol retained in a shaped body of silicone. Sustained release dosage forms comprising taxol and a pharmaceutical matrix carrier preferably comprise about 1 to about 70%, more preferably about 2 to about 50%, and even more preferably about 3 to about 8%, weight percent of taxol/weight percent of the total matrix carrier-therapeutic agent sustained release dosage form.

### Dosages, Formulation and Routes of Administration of the Therapeutic Agents

The amount of taxol administered is adjusted to treat vascular traumas of differing severity. For example, smaller doses are sufficient to treat lesser vascular trauma, e.g., to prevent vascular rejection following graft or transplant, while larger doses are sufficient to treat more extensive vascular trauma, e.g., to treat restenosis following angioplasty.

Administration of a taxol in accordance with the present invention may be continuous or intermittent, depending, for example, upon the recipient's physiological condition, whether the purpose of the administration is therapeutic or prophylactic, and other factors known to skilled practitioners. The administration of the dosage form of the invention may be essentially continuous over a preselected period of time or may be in a series of spaced doses, e.g., either before, during, or after procedural vascular trauma, before and during, before and after, during and after, or before, during and after the procedural vascular trauma. Moreover, the administration of the dosage form is selected so as to not further damage the traumatized vessel.

The local delivery of taxol can be by a variety of techniques which administer the agent at or near the traumatized vascular site. Examples of site-specific or targeted local delivery techniques are not intended to be limiting but to be illustrative of the techniques available. Examples include local delivery catheters, such as an infusion catheter, an indwelling catheter, or a needle catheter, stets, synthetic grafts, adventitial wraps, shunts and stents or other implantable devices, site specific carriers, direct injection, or direct applications.

Local delivery by an implant describes the surgical placement of a matrix that contains the therapeutic agent into the lesion or traumatized area. The implanted matrix releases the therapeutic agent by diffusion, chemical reaction, or solvent activators. See, for example, Lange, Science, 249, 1527 (1990).

An example of targeted local delivery by an implant is the use of a stent. Stents are designed to mechanically prevent the collapse and reocclusion of the coronary arteries or other vessels. Incorporation of a therapeutic agent into the stent can deliver the therapeutic agent directly to the lesion. Local delivery of agents by this technique is described in Koh, Pharmaceutical Technology (October, 1990).

For example, a metallic, plastic or biodegradable intravascular stent is employed which comprises the therapeutic agent. The stent preferably comprises a biodegradable coating, a porous or a permeable non-biodegradable coating, or a biodegradable or non-biodegradable membrane or synthetic graft sheath-like coating, e.g., PTFE, comprising the therapeutic agent. A more preferred embodiment of the invention is a coated stent wherein the coating comprises the sustained-release dosage form of taxol . In an alternative embodiment, a biodegradable stent may also have taxol impregnated therein, i.e., in the stent matrix.

A biodegradable stent with the therapeutic agent impregnated therein can be further coated with a biodegradable coating or with a porous non-biodegradable coating having the sustained release-dosage form of the therapeutic agent dispersed therein. This stent can provide a differential release rate of the therapeutic agent, i.e., there can be an initial faster release rate of the therapeutic agent from the coating, followed by delayed release of the therapeutic agent impregnated in the stent matrix, upon degradation of the stent matrix. The intravascular stent also provides a mechanical means of providing an increase in luminal area of a vessel.

Another example of targeted local delivery by an implant is the use of an adventitial wrap. The wrap comprises a pharmaceutically acceptable carrier matrix, e.g., a Pluronic gel which is free, or contained by a collagen mesh, which gel has dispersed therein a therapeutic agent. One embodiment of the invention is a pluronic gel (F-127, BASF) which is soluble at 4°C but solidifies at 37°C, e.g., on contact with fluid or tissue in a human. To prepare a pluronic gel containing wrap, 4 ml of phosphate buffer, pH 7.0 (Circ. Res, vol. 76, April 1995), was added to 1 g of pluronic gel F-127, which was mixed oversight at 4°C. Taxol was added to the mixture prior to local administration. The mixture may be applied directly to a surgically exposed artery wall, or may be applied to the surface of a bovine collagen mat (BioCore, Inc., Topeka, KS), which is then wrapped around the artery and the edges joined by sutures.

Another embodiment of the invention is the incorporation of taxol into the expanded nodal spaces of a PTFE (Impra, Inc., Tempe, AZ) vascular graft-like membrane which can surround, or be placed on the interior or on the exterior surface of, an interlumenal vascular stent, which comprises metal or a biodegradable or nonbiodegradable polymer. The sustained release dosage form fills the nodal spaces of the PTFE membrane wall and/or coats the inner and/or outer surfaces of the membrane.

Yet another embodiment of the invention is a mixture of a crystalline form of taxol in a bovine collagen gel (BioCore, Inc., Topeka, KS). Crystals varied in size from about 0.1 micron to about 1 mm. Generally, the crystals were pulverized to generate smaller sized crystals. This mixture is applied directly to the surface of the artery, and the surrounding subcutaneous tissues sutured around the vessel and the skin closed. Bovine collagen (BioCore, Inc., Topeka, KS) is dissolved in sterile saline (1:1) and the crystalline therapeutic agent added. Alternatively, the collagen gel is applied to a bovine collagen mesh which is then wrapped around the vessel and the edges sutured to hold the mesh in place. The bovine collagen mesh (BioCore, Inc.) is cut to size, e.g., 1 cm x 1 cm, and the therapeutic agent-collagen gel mixture is applied to the surface of the mesh.

A further embodiment of the invention comprises the entrapment of crystalline taxol in about a 0.1 to about 3, preferably about 0.5 to about 0.7, mm thick silicone membrane, e.g., silicone polymer Q-7 4840 (Dow Coming, Midland, MI). The polymer (part A and B, 1:1) is mixed with a spatula. An inert filler, e.g., mannitol, is powdered and sieved to a fraction 53-75 mesh size. Mannitol and taxol are mixed in predetermined proportions and then levigated with the polymer to form a composite. The composite is filled in a slab mold and compressed to 5000 psi. The composite is then cured at 80°C for 2 hours. The composite membrane is then cut to size, e.g., 1 cm x 1 cm, wrapped around the artery and held in place by suturing the membrane edges together.

Taxol may also be coated onto the exterior of the wrap. The wrap and/or the coating is preferably biodegradable.

Another example is the delivery by polymeric endoluminal sealing. This technique uses a catheter to apply a polymeric implant to the interior surface of the lumen. The dosage form of the invention is incorpated into the biodegradable polymer implant and is thereby released at the surgical site. This technique is described in PCT WO 90/01969 (Schindler, Aug. 23, 1989).

### Conditions Amenable to Treatment by the dosage form of the Invention

The dosage forms of the invention are useful to treat or inhibit a diminution in vessel lumen volume, area and/or diameter associated with a procedural vascular trauma. As used herein, "vessels" includes mammalian vessels, e.g., coronary vessels as well as peripheral, femoral and carotid vessels. It will be recognized that the dosage forms of the invention are not restricted in use for therapy following angioplasty; rather, the usefulness of the dosage forms will be proscribed by their ability to inhibit cellular activities of smooth muscle cells and pericytes in the vascular wall. Thus, a vascular trauma includes but is not limited to trauma associated with an interventional procedure, such as angioplasty, placement of a stent, shunt, stet, synthetic or natural graft, adventitial wrap, indwelling catheter or other implantable devices. Grafts include synthetic therapeutic agent-treated grafts, e.g., impregnated or coated grafts. well as an anti-proliferative or anti-migratory effect, can be achieved in a single dosing protocol.

The dosage form of the invention provides can be used in a method of treating a mammal having, or at risk of, diminution in vessel lumen volume, area or diameter, e.g., stenosis or restenosis of a blood vessel. The method comprises the administration of at least taxol in an amount effective to biologically stent a vessel, inhibit or reduce vascular remodeling of a vessel, inhibit or reduce vascular smooth muscle cell proliferation, or any combination thereof.

For the prevention of vessel lumen diminution associated with procedural vascular trauma, taxol can be administered before, during and/or after the procedure, or any combination thereof. For example, for the prevention of restenosis, a series of spaced doses of taxol is preferably administered before, during and/or after the traumatic procedure (e.g., angioplasty). The dose may also be delivered locally, via an implantable device, e.g., a catheter, introduced into the afflicted vessel during the procedure. Preferably, the sustained release dosage form is administered via the implantable device during the traumatic procedure. After the traumatic procedure is conducted, a series of follow-up doses can be administered over time, preferably in a sustained release dosage form, for a time sufficient to substantially reduce the risk of, or to prevent, restenosis. A preferred therapeutic protocol duration for this purpose involves administration from about 3 to about 26 weeks after angioplasty.

It will be recognized by those skilled in the art that therapeutically/prophylactically effective dosages of taxol will be dependent on several factors, including, e.g.: a) the atmospheric pressure and duration of the pressure applied during infusion; b) the time over which taxol administered resides at the vascular site; c) the nature of the vascular trauma and therapy desired; d) for sustained release dosage forms, the rate of release of the therapeutic agent from the dosage form, and/or e) for sustained release dosage forms, the intercellular and/or intracellular localization of the dosage form. Those skilled practitioners trained to deliver drugs at therapeutically effective dosages (e.g., by monitoring drug levels and observing clinical effects in patients) will determine the optimal dosage for an individual patient based on experience and professional judgment. Those skilled in the art will recognize that infiltration of taxol into the intimal layers of a traumatized vessel wall in free or sustained release dosage form is subject to variation and will need to be determined on an individual basis.

A therapeutically effective dosage of taxol will be typically reached when the concentration of taxol in the fluid space between the balloons of the catheter is in the range of about 10⁻³ to 10⁻¹²M. It will be recognized from the Examples provided herewith that therapeutic agents and dosage forms of the invention may only need to be delivered in an anti-proliferative therapeutic dosage sufficient to expose the inner 10%, preferably the inner 20%, and more preferably the inner 99%, of the tunica media cells lining the lumen to the therapeutic agent. This dosage can be determined empirically, e.g., by a) infusing vessels from suitable animal model systems and using immunohistochemical, fluorescent or electron microscopy methods to detect the agent and its effects ; and b) conducting suitable in vitro studies.

For example, with respect to catheter delivery, it will be recognized by those skilled in the art that therapeutically/prophylactically effective dosages of the therapeutic agents and dosage forms will be dependent on factors including: a) the atmospheric pressure applied during infusion; b) the time over which the agent administered resides at the vascular site; c) the form of the therapeutic or prophylactic agent employed; and/or d) the nature of the vascular trauma and therapy desired. Catheters which may be useful in the practice of the invention include catheters such as those disclosed in Just et al. (U.S. Patent No. 5,232,444), Abusio et al. (U.S. Patent No. 5,213,576), Shapland et al. (U.S. Patent No. 5,282,785), Racchini et al. (U.S. Patent No. 5,458,568), Wolinsky (U.S. Patent No. 4,824,436), Spears (U.S. Patent No. 4,512,762) and Shaffer et al. (U.S. Patent No. 5,049,132), the disclosures of which are incorporated by reference herein.

A therapeutically effective dosage is generally the pericellular agent dosage in smooth muscle cell tissue culture, i.e., a dosage at which at a continuous exposure results in a therapeutic effect between the toxic and minimal effective doses. This therapeutic level is obtained *in vivo* by determining the size, number and taxol concentration and release rate required for particles infused between the smooth muscle cells of the artery wall to maintain this pericellular therapeutic dosage. The dosage form should release the therapeutic agent at a rate that approximates the pericellular dose of from about 0.001 to about 100 micrograms/ml taxol.

The invention will be better understood by making reference to the following specific examples.

### EXAMPLE 17

### Sustained Release Formulations of Cytochalasin B and Taxol

To determine the efficacy of the local, sustained release dosage forms of cytochalasin B or taxol to inhibit restenosis, cytochalasin B or taxol in a supporting structure, e.g., a "wrap," was applied to the adventitial tissue surrounding a balloon traumatized rabbit carotid artery (Groups 1-11) or a balloon traumatized pig femoral artery (Group 12) (Table 16).

The arteries in the animals in Group 1a and 1b were treated with 20 mg of cytochalasin B in 1 g of a bovine collagen gel (BioCore, Inc., Topeka, KS) that was supported by, or enclosed in, a bovine collagen mesh wrap (Skin Temp-Biosynthetic Skin Dressing, BioCore, Inc., Topeka, KS). At 1 week post treatment, the cytochalasin B treated artery in animal 1233 showed no intimal or adventital proliferation. There was marked cell death in the outer zone of the tunica media with heterophils infiltrating the tunica media. Heterophils were present outside the wrap, but cytochalasin B inhibited heterophils and macrophages from infiltrating the wrap. The artery of the control (1249) animal had moderate intimal proliferation, and heterophils and histiocytes were infiltrating into the wrap. Cell death in the tunica media was minimal.

At 2 weeks post-treatment, there was minimal intimal and adventital proliferation in the cytochalasin B wrap-treated area of the artery (animal 1224). The intima was loosely arranged and there was minimal heterophil infiltration. Syncytial giant cells were present. In the artery of the control wrap animal (animal 1222), there was moderate intimal proliferation with heterophils and macrophage in the wrap area. These cells were visible in the tunica media.

At three weeks post-treatment, there was no intimal proliferation observed in the cytochalasin B wrap-treated area of the artery (1244). Heterophils and syncytial giant cells were present around the wrap. There was significant necrosis of the cells in the tunica media with infiltrating heterophils and macrophages. No endothelium was present. In the control (animal 1232) artery, there was marked intimal proliferation, with well organized adventitia and perivascular tissue. Heterophils and macrophages were infiltrating the wrap. The cells in the tunica media were viable and there was a mural thrombus in the vessel lumen. Thus, inhibition of intimal proliferation was seen in the arteries of Group 1a and 1b animals treated with cytochalasin B, however, there was significant reaction to the wrap material.

The arteries in the animals in Group 2a and 2b were treated with cytochalasin B (30% wt/wt; 300 mg cytochalasin B/g silicone) in a silicone wrap (Q-7 4840, Dow Corning, Midland, MI). One week post-trauma there was no significant intimal or adventitial proliferation of smooth muscle cells (SMCs) or mesenchymal tissue (animal 1229). There was significant necrosis of the SMCs in the outer zone of the tunica media. In areas that appeared to have been minimally traumatized by the torquable ballon there was minimal to no cellular necrosis. This indicated that traumatized cells were more prone to die when exposed to this dose of cytochalasin B but that this dose was not cytocidal to minimally traumatized or normal SMCs. There was minimal mononuclear and polymorphoneuclear cell infiltration into the tunica media. A few heterophils were seen infiltrating from the vessel lumen.

In the control animal (1228), there was less cellular necrosis in the tunica media, and the necrosis present was located in the inner zone rather than the outer zone of the artery wall. Thus, cytochalasin B inhibition of cellular repair appears to increase tunica media necrosis. The control also lacked tunica media or adventitial proliferation and organization of the perivascular clot. Cellular infiltration in any area was minimal.

Two weeks after initiating cytochalasin B treatment there was complete inhibition of intimal proliferation and only minimal perivascular clot organization which was primarily due to fibrin formation and not mesenchymal proliferation (animal 1227). There was mild infiltration of polymorphonuclear cells and minimal infiltration of mononuclear cells into the tunica media and adventitia. No endothelium was present in the wrap area, except for a few small isolated foci. The control artery (animal 1226) had moderate intimal proliferation and adventitial proliferation with mesenchymal organization of the perivascular clot area. Foci of endothelial proliferation were larger and more extensive in the control animal compared to the cytochalasin B treated vessel.

With 3 weeks exposure (animal 1212) to cytochalasin B in a silicone wrap, the vessel showed marked cell loss in the tunica media which was most severe in the outer zone. Cellular infiltration in the tunica media and adventitia was minimal and endothelilzation was only present in a few focal areas. There was moderate, irregular intimal proliferation; however, the intimal cells and what few endothelial cells that were present were unorganized and lack polarity. The inhibition of migration by cytochalasin B resulted in this loss of organization or polarity. The intimal proliferation was also mild in the control vessel (1230); however, the intima was well organized and was almost completely endothelized. There was mininal cell loss from the tunica media. Thus, significant intimal inhibition was seen in the first two weeks in Group 2 treated animals.

The vessels in the animals in Group 3a and 3b were treated with 8 mg cytochalasin B in 100 mg of a pluronic gel (F-127, BASF) that was supported by a 1 cm x 1 cm bovine collagen mesh wrap. One week after treatment, the cytochalasin B treated artery of animal 1250 had mild intimal proliferation that was irregular in thickness. There was approximately 30% re-endothelization and the tunica media cells were viable with the most significant loss (mild) being in the inner zone of the tunica media. There was a marked pyogranulomatous reaction to the pluronic gel in the perivascular and adventitial region. Complete thrombosis of the control artery from animal 1261 prevented its evaluation.

At two weeks, the pluronic gel with cytochalasin B stimulated a marked pyogranulomatous reaction in the adventitial and perivascular tissue. There was mild, irregular intimal proliferation and complete endothelization. The tunica media cells were viable. There appeared to be a mild cell loss from the inner zone of the tunica media. The artery of the control animal (1247) had mild, irregular intimal proliferation, complete endothelization with plump endothelial cells and viable cells in the tunica media. There was marked pyogranulomatous inflammatory reaction to reminants of the pluronic gel.

At three weeks, the arteries of animals 1248 and 1246 showed reminants of the collagen wrap; however, the wrap was less resolved in the cytochalasin B treated animal (1248) than in the control (1246). This retardation of wrap resorbsion may result from cytochalasin B inhibition of macrophage migration and function. Both the treated and control had a moderate amount of intimal hyperplasia at 3 weeks, so there was no significant amount of intimal inhibition by cytochalasin B when administered in the pluronic gel. Foci of dystrophic mineralization were seen in the cytochalasin B treated artery. Thus, no inhibitory effect on the intima was observed in these animals (Group 3) at 1, 2 or 3 weeks after the initiation of treatment.

The arteries of the animals in Group 4a and b that were treated with 100 mg cytochalasin B (10% wt/wt) in a 1 g silicone wrap had significant inhibition of intimal proliferation at all time points. In the cytochalasin B wrap-treated artery of animal 1259, there was no intimal proliferation or adventitial fibrosis present at one week after treatment. Ectatic vessels were present in the adventitia and the perivascular clot was unorganized and composed of only fibrin. There was marked cell loss from the tunica media, especially in the outer zone. Heterophils were seen infiltrating the tunica media. In the control artery (1206), there was no intimal proliferation at 1 week; however, there was early fibrous organization of the perivascular clot. Cellular loss from the tunical media was more diffuse than in the cytochalasin B wrap which was most severe in the outer zone.

The cytochalasin B wrap-treated artery of animal 1253 had minimal intimal proliferation at two weeks, compared to the control (1258) wrap-treated artery which had maximal intimal proliferation. The intimal proliferation in the cytochalasin B wrap-treated artery was irregular and appeared to be the result of organizing mural thombi by infiltrating SMC. There was only loose thin layers of platelets in the cytochalasin B wrap-treated artery, with margination of heterophils. There was no endothelization in the cytochalasin B wrap-treated artery and <20% endothelization in the control artery. The perivascular clot was unorganized and remained fibinous in the cytochalasin B wrap-treated artery and well organized in the control artery. The control artery had minimal cell loss from the tunica media while the cytochalasin B wrap-treated artery had marked cell loss.

At three weeks, the cytochalasin B wrap-treated artery (1251) showed minimal to no intimal proliferation. The intimal proliferation appeared to occur where there was less cell loss from the tunica media. There was early re-endothelization in the cytochalasin B wrap-treated artery, but the cells were often rounded, loosely attached and only a few scattered foci were present (<10%). The perivascular clot in the cytochalasin B wrap-treated artery was unorganized and still consisted of fibrin, whereas the control artery was well organized with fibroblasts and collagen matrix. The control artery was completely thrombosed, there was marked initmal production in distal areas of the thrombus which were less completely organized.

The arteries in the animals in Group 5a and b were treated with 50 mg taxol in 1 g of a silicone wrap (5% wt/wt). This treatment showed marked inhibition of intimal proliferation at all time points. The taxol wrap-treated artery (animal 1278 at 1 week) had no intimal or adventitial proliferation and the perivascular clot was fibrinous and unorganized. There was a marked loss of tunica media cells and no endothelial lining present. The control (1279) had mild intimal proliferation with very early fibrosis of the fibrinous perivascular clot. The lumen was approximately 85% re-endothelized. Both the treated and control arteries had mild heterophil infiltration into the tunica media and adventitia.

The artery from animal 1281, which had been treated for at two weeks with taxol, had no intimal proliferation, minimal adventitial fribrosis and marked cell necrosis in the tunica media with mild heterophil infiltration. Focal areas of necrosis and dystrophic mineraliztion were present in the adventitia and perivascular clot tissue. The artery from the control animal (1280) had moderate intimal proliferation, with marked organization of the adventitia and perivascular clot. The lumen was 100% re-endothelized and the tunica media SMCs were viable in the control artery.

At three weeks, the taxol wrap-treated artery (1242) had no intimal proliferation and was 50% re-endothelized with plump appearing endothelial cells. There was minimal organization of the perivascular clot and marked cell loss from the tunica media. There was mild infiltration of heterophils into the tunica media and marginating on the vessel lumenal surface. The control artery (1234) had marked intimal proliferation and fibrosis of the adventitia and perivascular clot. Cells in the tunica were viable.

In Group 6a and 6b, taxol-treated arteries also had a marked inhibition 2 weeks after the wrap was removed. Animal 1276 had a taxol wrap for 2 weeks, then the wrap was removed and the animal sacrificed 3 weeks later. Following the 3 week recovery period from the taxol wrap removal (1276) there was only minimal intimal proliferation, except in a few focal areas that appeared to be thickened due to SMC organization of mural thrombi, in this artery. The adventitia was well organized and there was a significant cell loss in the tunica media but the cells present were viable. The lumen was approximately 90% re-endothelized. The control (1277) artery had marked intimal proliferation, well organized perivascular and adventical tissue and was 100% re-endothelized.

The results observed for Group 7a animals demonstrated that cytochalasin B-treated arteries (10% wt/wt) showed no intimal proliferation for 2 weeks. The decrease in the release rate of the cytochalasin B, however, resulted in a mild intimal proliferation by week three after wrap placement. At one week (1257), the arteries showed no intimal proliferation, and a marked necrosis of tunical media SMCs with moderate heterophil infiltration. There was no endothelium and heterophils and macrophages were marginated along the lumen surface. Moreover, there was no evidence of platelet aggregates adhering to vessel wall. At two weeks (1265), the arteries were similar morphologically to the one week arteries. By three weeks (1266), the arteries showed mild irregular intimal proliferation. Furthermore, heterophils were rare in the tunica media, the lumen was 70% re-endothelized and there was early fibrosis in the adventitia with unorganized perivascular clot still present, in the treated arteries. This indicated that by 3 weeks the level of therapeutic agent had fallen below therapeutic level within the artery wall; however, there was still enough drug to have an inhibitory effect on clot organization immediately adjacant to the wrap.

The arteries of Group 8a animals, which were treated with 10% cytochalasin B for 2 weeks, then the wrap was removed and vessels evaluated 2 weeks later, had variable intimal proliferation within and between animals. The artery of animal 1254 had variable intimal proliferation which ranged from none to mild, well developed adventitial fibrosis, marked cell loss in the tunic media and focal areas of dystrophic mineralization in the outer tunica media and adventitia. The mild intimal proliferation areas were at the ends of the wrap area, suggesting an infiltration from the adjacent untreated artery regions. The lumen was approximately 60% re-endothelized. The artery of animal 1255 had mild to moderate intimal proliferation, viable cells in the tunica media and well organized tissue in the adventitia. The lumen was 100% re-endothelized. The artery in animal 1256 was completely thrombosed. Proximal to the chronic thrombus in the area of the wrap was an acute thrombus and there was moderate intimal proliferation.

While there was moderate intimal proliferation in the arteries of some animals, the proliferation in these arteries was still less than the controls in Group 9b. The mannitol control silicone wraps were on the artery for two weeks following balloon trauma and then removed and the animal necropsied and the artery histologically evaluated 1 week following wrap removal. Two of the arteries (1267 and 1268) had moderate intimal proliferation with 100% re-endothelization and one had maximum proliferation. The one with maximum intimal prolifertion had an acute occluding thrombus present.

The arteries in the animals in Groups 10 and 11 were treated with 10 mg cytochalasin B loaded in 1 g of a silicone wrap (1% wt/wt) that was applied to the artery for 2 weeks, surgically removed, and histologically evaluated 2 or 4 weeks later, respectively. No significant difference was seen by qualitative evaluation between the test and control animals. Animals 1304 and 1305 had a cytochalasin B (1%) wrap for 2 weeks which was then removed. Two weeks after the removal the animal was sacrificed. The artery from animal 1304 showed moderate initmal proliferation in most areas of the wrap, in areas of marked tunica media cell necrosis and wall dystrophic mineralization the proliferation was mild. There was 100% re-endothelization and no heterophils were present in the intima or tunica media. The adventitia and perivascular clot area was well organized. The artery from animal 1305 was similar to the artery from animal 1304 morphologically. The artery from animal 1306 showed marked intimal proliferation, no infiltrating heterophils in the intima or tunica media and was 100% re-endothelized.

Animals 1307, 1308 and 1309 were exposed to a cytochalasin B (1%) wrap for 2 weeks which was then removed. Four weeks after removal the animals were sacrificed. The artery from animal 1307 had moderate initmal proliferation with focal areas of thickening due to mural thrombus organization by SMCs. There was significant loss of cells from the tunica media and the elastic elamina appear collapsed. A few heterophils were present in the adventitia. There were areas or sections in the wrap area with minimal intimal proliferation. The artery from animal 1308 showed moderate intimal proliferation with areas of marked cell loss in the tunica media and dystrophic minearalization in the outer zone of the tunica media. The vessel was 100% re-endothelized. The artery from animal 1309 had marked intimal proliferation with a well organized aventiticia and perivascular region. Animal 1311 was not evaluated due to thrombosis. The results of the artery from animal 1312 were quite variable, with sections showing a range of intimal proliferation, from mild to moderate. Endothelization appeared to be complete in these arteries.

The arteries from Group 12 animals (pig femoral arteries) that were treated with 30% wt/wt cytochalasin B loaded silicone wraps showed significantly inhibited intimal proliferation for the first two weeks. While there was intimal proliferation in the arteries 3 weeks later, the proliferation was still less than the proliferation observed for the controls.

**TABLE 16**

| ANIMAL # | GROUP # | TREATMENT | SURGERY DATE | NECROPSY DATE | TIME POST TREATMENT | GRADE |
|---|---|---|---|---|---|---|
| 1233 | 1a | CytoB+Bovine col gel+col mat | 2/28/96 | 3/7/96 | 1 wk | 1 |
| 1249 | 1b | Control+Bovine col gel+col mat | 2/28/96 | 3/7/96 | 1 wk | 3 |
| 1224 | 1a | CytoB+Bovine col gel+col mat | 2/28/96 | 3/15/96 | 2 wks | 2 |
| 1222 | 1b | Control+Bovine col gel+col mat | 2/28/96 | 3/15/96 | 2 wks | 3 |
| 1244 | 1a | CytoB+Bovine col gel+col mat | 2/28/96 | 3/20/96 | 3 wks | 1 |
| 1232 | 1b | Control+Bovine col gel+col mat | 2/28/96 | 3/20/96 | 3 wks | 3 |
| | | | | | | |
| 1229 | 2a | CytoB 30%+silicone | 2/22/96 | 2/28/96 | 1 wk | 0 |
| 1228 | 2b | Control + silicone | 2/22/96 | 2/28/96 | 1 wk | 2 |
| 1227 | 2a | CytoB 30%+silicone | 2/22/96 | 3/7/96 | 2 wks | 0 |
| 1226 | 2b | Control + silicone | 2/22/96 | 3/7/96 | 2 wks | 3 |
| 1212 | 2a | CytoB 30%+silicone | 2/22/96 | 3/15/96 | 3 wks | 2 |
| 1230 | 2b | Control + silicon | 2/22/96 | 3/15/96 | 3 wks | 2 |
| 1250 | 3a | CytoB+pluronic gel+col. wrap | 3/7/96 | 3/15/96 | 1 wk | 2 |
| 1261 | 3b | Control+pluronic gel+col. wrap | 3/7/96 | 3/15/96 | 1wk | NA |
| 1245 | 3a | CytoB+pluronic gel+col. wrap | 3/7/96 | 3/20/96 | 2 wks | 2 |
| 1247 | 3b | Control+pluronic gel+col. wrap | 3/7/96 | 3/20/96 | 2 wks | 2 |
| 1248 | 3a | CytoB+pluronic gel+col. wrap | 3/7/96 | 3/28/96 | 3 wks | 3 |
| 1246 | 3b | Control+pluronic gcl+col. wrap | 3/7/96 | 3/28/96 | 3 wks | 3 |
| | | | | | | |
| 1259 | 4a | CytoB 10%-mannitol+silicone | 4/8/96 | 4/15/96 | 1 wk | 0 |
| 1260 | 4b | Control-marmitol+silicone | 4/8/96 | 4/15/96 | 1 wk | 0 |
| 1253 | 4a | CytoB 10%-mannitol+silicone | 4/8/96 | 4/22/96 | 2 wks | 1 |
| 1258 | 4b | Control-mannitol+silicone | 4/8/96 | 4/22/96 | 2 wks | 4 |
| 1251 | 4a | CytoB 10%-mannitol+silicone | 4/8/96 | 4/29/96 | 3 wks | 0 |
| 1252 | 4b | Ccntrol-mannitol+silicone | 4/8/96 | 4/29/96 | 3 wks | 4 |
| | | | | | | |
| 1278 | 5a | Taxol + silicone | 3/20/96 | 3/2//96 | 1 wk | 0 |
| 1279 | 5b | Control-silicono | 3/20/96 | 3/28/96 | 1 wk | 2 |
| 1281 | 5a | Taxol + silicone | 3/20/96 | 4/4/96 | 2 wks | 1 |
| 1280 | 3b | Control-silicone | 3/20/96 | 4/4/96 | 2 wks | 3 |
| 1242 | 5a | Taxol + silicone | 3/7/96 | 3/28/96 | 3 wks | 0 |
| 1243 | Sb | Control-silicone | 3/7/96 | 3/28/96 | 3 wks | 4 |
| | | | | | | |
| 1276 | 6a | Taxol + silicone | 3/20/96 | 4/23/96 | 2 wks - 3 wks | 1 |
| 1277 | 6b | Control-silicone | 3/20/96 | 4/23/96 | 2 wks - 3 wks | 4 |
| | | | | | | |
| 1237 | 7a | Cytob 10%-mannitol=silicone | 5/16/96 | 5/23/96 | 1 wk | 0 |
| 1265 | 7a | Cytob 10%-mannitol=silicone | 5/16/96 | 5/29/96 | 2 wks | 0 |
| 1266 | 7a | Cytob 10%-mannitol=sllicone | 5/16/96 | 6/5/96 | 3 wks | 2 |
| | | Note: see control cases | 1260=1wk | 1258=2wks | 1252=3wks | |
| 1254 | 8a | CytoB 10%-mannitol+silicone | 5/16/96 | 6/11/96 | 2 wks- 2 wks | 0-2 |
| | | | | | | |
| 1255 | 8a | CytoB 10%-mannitol+silicone | 5/16/96 | 6/11/96 | 2 wks - 2 wks | 2-3 |
| 1256 | 8a | CyoB 10%-mannitol+silicone | 5/16/96 | 6/11/96 | 2 wks-2 wks | 3 |
| | | | | | | |
| 1267 | 9b | Control-mannitol+silicone | 3/23/96 | 6/4/96 | 2 wks - 1 wks | 3 |
| 1268 | 9b | Control-mannimi+silicone | 5/23/96 | 6/14/96 | 2 wks- 1 wks | 3 |
| 1269 | 9b | Control-nmunitol+silicone | 5/23/96 | 6/14/96 | 2 wks - 1 wks | 4 |
| | | | | | | |
| 1304 | 10a | CytoB 1%-mannitol+silicone | 6/10/96 | 7/9/96 | 2 wks-2 wks | 3 |
| 1305 | 10a | CytoB 1%-mannitol+silicone | 6/10/96 | 7/9/96 | 2 wks-2wks | 3 |
| 1306 | 10a | CyrtoB 1%-mannitol+siliconce | 6/10/96 | 7/9/96 | 2 wks - 2 wks | 4 |
| 1307 | 11a | CyoB 1%mannitol+silicone | 6/10/96 | 7/22/96 | 2 wks -4 wks | 3 |
| 1308 | 11a | CytoB 1%-mannitol+silicone | 6/10/96 | 7/22/96 | 2 wks - 4 wks | 3 |
| 1309 | 11a | CytoB 1%-mannitol+silicone | 6/10/96 | 7/22/96 | 2 wks - 4 wks | NA |
| 1310 | 11b | Control-mannitol+silicone | 6/10/96 | 7/22/96 | 2wks - 4wks | 4 |
| 1311 | 11b | Control-mannitol+silicone | 6/10/96 | 7/22/96 | 2wks - 4wks | NA |
| 1312 | 11b | Control-mannitol+silicone | 6/10/96 | 7/22/96 | 2 wks - 4 wks | 3 |
| 1029LF | 12e | CytoB 30%-silicone wrap | 2/1/96 | 2/22/96 | 3 wks. | 2 |
| 1020RF | 12f | Control silicone wrap | 2/1/96 | 2/22/96 | 3 wks. | 3 |
| 1030LF | 12e | CytoB 30%-silicone wrap | 2/7/96 | 2/22/96 | 2 wks. | 1 |
| 1030RF | 12d | Control silicone wrap | 2/7/96 | 2/22/96 | 2 wks. | 4 |
| 1036LF | 12a | CytoB 30%-silicone wrap | 2/1/96 | 2/7/96 | 1 wt. | 1 |
| 1036RF | 12b | Control silicone wrap | 2/1/96 | 2/7/96 | 1 wk. | 1 |

In summary, intimal proliferation of traumatized pig arteries was significantly inhibited with both cytochalasin B and taxol in sustained release dosage form. The best controlled sustained release of therapeutic agent, without the stimulation of secondary inflammatory reaction, was obtained with an adventitial wrap material comprising silicone. The silicone wraps inhibited intimal proliferation with 30% and 10% loadings of cytochalasin B; however, as the level of release drops offbetween 2 and 3 weeks there was initiation ofintimal proliferation. When wraps were left in place for 2 weeks then surgically removed and the arteries examined from 1 to 4 weeks later, there appeared to be an intimal proliferation rebound effect. The rebound effect occurred when the intimal proliferation of the artery treated with the therapeutic agent approaches, but is still less than, the intimal proliferation in the control artery. The animal treated with taxol appeared to have less of a rebound effect than the cytochalasin B treated arteries.

### EXAMPLE 18

### Delivery of Crystalline Cytochalasin B or Taxol

The *in vivo* tissue distribution of cytochalasin B administered in crystalline form was evaluated in balloon traumatized swine femoral arteries after local delivery. A femoral artery of a Yorkshire crossbred swine was balloon traumatized by overinflation and rotation of a Vascu-Flo™ Silicone embolectomy catheter. Balloon trauma was immediately followed by intravascular delivery of 10 µg/ml ³H-cytochalasin B crystals (Sigma Chemical Co., St. Louis, MO) in saline (saturated) for three minutes under 1 atm of pressure. Blood flow was resumed in the artery for five minutes prior to sacrifice of the animal. An analysis of the tissue distribution of ³H-cytochalasin B showed that this method was effective at delivering 31 ug of ³H-cytochalasin B which localized predominantly to the adventitia. ³H-cytochalasin B was visualized histologically by the presence of silver grains in an autoradiographic emulsion. Thus, these results showed that crystalline cytochalasin B can be delivered locally to a vessel wall *in vivo*.

Another study employed twenty, male, Sprague-Dawley rats. The rats underwent balloon trauma to their left carotid artery, followed by inter-arterial infusion of a solution containing 1 mg crystalline cytochalasin B in 300 ml vehicle (Hanks sterile salt solution with 0.5% Cremophor) or a diluent (saline) control. Animals were sacrificed immediately after infusion, and 2, 4, 7 and 14 days post-trauma and infusion. Post-sacrifice, the left and the right (control) carotid arteries were removed. Samples of arteries were obtained for quantitation of ³H-cytochalasin B by oxidation and scintillation counting, histopathology, autoradiography and vascular morphometry. Histopathology documented uniform, circumferential balloon trauma in the arterial wall of the left carotid arteries.

Autoradiographically, cytochalasin B crystals were present on day 0 in intraluminal fibrin clots, adherent to the intima but rarely present in the adventitia. By day 2, the number of crystals diminished compared to day 0, and by day 4 crystals were not detectable by autoradiography. The autoradiographic results correlated closely with quantitative assessment of ³H-cytochalasin B by oxidation and scintillation counts in which approximately 8 ug of cytochalasin B was present over the treated length of artery on day 0 and slightly less than 2 ng was present by day 2. However, one of the two animals sacrificed on day 4 still had cytochalasin B levels above background. Morphometric analysis of left carotid arteries of crystalline cytochalasin B treated rats compared to diluent treated rats showed no statistically significant reduction in neointima formation. However, the five treated rats had a higher mean luminal area and a smaller neointimal area than diluent treated control.

Cytochalasin B and taxol were administered periadventially. Three groups of seven adult male rats underwent balloon trauma of the left carotid artery immediately followed by periadventitial placement of either cytochalasin B crystals (7.8 - 11.8 mg/rat), taxol crystals (3.4 - 6.5 mg/rat), or no drug (control). The cytochalasin B and taxol crystals were placed in a uniform pattern which covered the surgically exposed surface of the carotid artery, followed by closure of surrounding subcutaneous skin and tissues by sutures. Fourteen days later rats were sacrificed and their carotid arteries processed for histologic and morphometric analysis.

Two cytochalasin B treated animals died due to acute hemorrhage at the surgical site and hypovolemic shock prior to the 14 day sacrifice point. Two additional cytochalasin B treated and one taxol treated animal were sacrificed with rapidly enlarging subcutaneous swelling and hemorrhage at the surgical site prior to the 14 day sacrifice point. All animals treated with either cytochalasin B or taxol crystals had significant toxicity at the surgical site which was characterized by varying degrees of hemorrhage, necrosis of the vessel wall, necrosis of adjacent skeletal muscle and inflammation. In addition, both the taxol and cytochalasin B treated animals had a delay in post-surgical weight gain.

The three cytochalasin B treated, 6 taxol treated and 7 control animals which survived to the 14 day sacrifice point were evaluated morphometrically. Taxol treated animals had statistically significantly larger luminal areas and no neointimal proliferation when compared to the balloon traumatized, untreated control animals in a two-tailed t-test with p < 0.05. Cytochalasin B treated animals showed no statistical difference from the controls in luminal area, neointimal area, medial area, areas bounded by the internal and external elastic lamina or intimal to medial ratio.

To further evaluate the efficacy of crystalline taxol to inhibit neointimal formation in rats, four groups of 5-6 adult male rats underwent balloon trauma of the left carotid artery followed immediately by periadventitial delivery of 1, 0.1, 0.01 or 0 mg of taxol crystals in 500 mg of a pluronic polymer in gel matrix. Fourteen days later, the rats were sacrificed, serum was collected and their carotid arteries were processed for histologic and morphometric analysis.

Five animals (3 -1 mg and 2 - 0.01 mg) died post-surgically due to technical difficulties. Grossly, myonecrosis of the adjacent skeletal muscle (pale white regions of the musculature) was present in 3/3, 1/5, 0/4 and 0/5 animals in the 1 mg, 0.1 mg, 0.01 mg and control groups, respectively. Histologically, myonecrosis was confirmed in the adjacent skeletal muscle and in some regions of the tunica media of the left carotid artery in the 1 mg treatment group but not in the other groups. Morphometrically, there was no statistical significance in luminal area, neointimal area, area bounded by the internal elastic lamina, area of the tunica media, area bounded by the external elastic lamina or neointimal/medial ratio when compared by analysis of variance using the excell data analysis software package.

Periadventitial treatment of rat carotid arteries with 1 mg taxol crystals in 500 mg of a pluronic gel resulted in gross myonecrosis of the adjacent musculature. While the number of animals surviving in this group was too low to assess for statistical significance in the reduction of neointimal formation, neointimal area was 38% less than that of control animals.

For animals treated with 0.1 and 0.01 mg taxol, a reduction in their neointimal area and neointimal/medial ratio was observed when compared to control animals, although this did not reach statistical significance given the small number of animals per group. Moreover, animals in the lower dose groups showed no (0.01 mg), minimal (0.1 mg) or limited (1.0 mg) toxicity, indicating that lower doses may be efficacious and exhibit fewer adverse side effects than doses greater than 1.0 mg.

## Claims

1. A dosage form comprising a taxol for use in inhibiting or reducing diminution in vessel lumen area of a mammalian blood vessel, wherein the taxol is in substantially crystalline form and wherein the crystal size of the taxol is 0.1 micron to 1 mm.

2. The dosage form of claim 1 wherein the crystal size is 1 micron to 25 micron.

3. The dosage form of claims 1 or 2 wherein taxol is administered via an implantable device.

4. The dosage form of claim 3 wherein taxol is releasably embedded in, coated on, or embedded in and coated on, the implantable device.

5. The dosage form of claim 3 wherein taxol is releasably embedded in microparticles, nanoparticles, or a mixture thereof.

6. The dosage form of claim 5 wherein the microparticles or nanoparticles are releasably embedded in, coated on, or embedded in and coated on, the implantable device.

7. The dosage form of one of claims 3 to 6 wherein the device is an adventitial wrap, an artificial graft, a catheter, a stent or a shunt.

8. The dosage form of one of claims 3 to 6 wherein the implantable device is a catheter which is implanted for a limited amount of time.

9. The dosage form of claims 1 or 2 wherein taxol is releasably embedded in coated on, or embedded in and coated on, a non-liquid matrix.

10. The dosage form of one of the preceeding claims wherein the administration is before, during or after procedural vascular trauma, or any combination thereof.

11. The dosage form of one of the preceeding claims wherein the dosage form is delivered locally by a catheter which is introduced into the afflicted vessel during the procedure.

12. The dosage form of one of the preceeding claims wherein the vessel is traumatized by angioplasty, placement of a stent, placement of a shunt, or natural grafting, or any combination thereof.

13. The dosage form of claim 11 wherein the trauma shock is resulting from vessel overstretching by a balloon catheter during angioplasty.

14. The dosage form of one of the preceeding claims wherein the administration is local.

15. The dosage form of one of the preceeding claims wherein the vessel is procedurally traumatized.

16. Crystalline taxol having a crystal size of 0.1 micron to 1 mm for use in inhibiting or reducing diminution in vessel lumen volume in a procedurally traumatized mammalian blood vessel.

17. Crystalline taxol having a crystal size of 0.1 micron to 1 mm for use in inhibiting or reducing diminution in vessel lumen volume in a procedurally traumatized mammalian blood vessel, wherein taxol is administered *in situ* by means of an implantable device wherein taxol is releasably embedded in, coated on or embedded in and coated on the implantable device.

## Patentansprüche

1. Dosierungsform, umfassend eine Taxol, zur Verwendung bei der Inhibierung oder Verringerung der Abnahme der Gefäßlumenfläche eines Säugetierblutgefäßes, wobei das Taxol in im Wesentlichen kristalliner Form vorliegt und wobei die Kristallgröße des Taxols 0,1 µm bis 1 mm beträgt.

2. Dosierungsform nach Anspruch 1, wobei die Kristallgröße zwischen 1 µm und 25 µm liegt.

3. Dosierungsform nach Anspruch 1 oder 2, wobei das Taxol über eine implantierbare Vorrichtung verabreicht wird.

4. Dosierungsform nach Anspruch 3, wobei das Taxol in wiederfreisetzbarer Form in die implantierbare Vorrichtung eingebettet ist, darauf aufgeschichtet ist, oder darin eingebettet und darauf aufgeschichtet ist.

5. Dosierungsform nach Anspruch 3, wobei das Taxol in wiederfreisetzbarer Form in dieMikroteilchen, Nanoteilchen, oder Mischungen davon, eingebettet ist.

6. Dosierungsform nach Anspruch 5, wobei die Mikroteilchen oder die Nanoteilchen in wiederfreisetzbarer Form in die implantierbare Vorrichtung eingebettet sind, darauf aufgeschichtet sind, oder darin eingebettet und darauf aufgeschichtet sind.

7. Dosierungsform nach einem der Ansprüche 3 bis 6, wobei die Vorrichtung ein adventitieller Umschlag, ein künstliches Transplantat, ein Katheter, ein Stent oder ein Shunt ist.

8. Dosierungsform nach einem der Ansprüche 3 bis 6, wobei die implantierbare Vorrichtung ein Katheter ist, welcher für einen begrenzten Zeitraum implantiert ist.

9. Dosierungsform nach Anspruch 1 oder 2, wobei das Taxol in wiederfreisetzbarer Form in eine nichtflüssige Matrix eingebettet ist, darauf aufgeschichtet ist, oder darin eingebettet und darauf aufgeschichtet ist.

10. Dosierungsform nach einem der vorstehenden Ansprüche, wobei die Verabreichung vor, während oder nach einem durch einen Eingriff verursachtem vaskulären Trauma, oder einer Kombination davon, erfolgt.

11. Dosierungsform nach einem der vorstehenden Ansprüche, wobei die Dosierungsform lokal durch einen Katheter verabreicht wird, der während des Eingriffs in das befallene Gefäß eingeführt ist.

12. Dosierungsform nach einem der vorstehenden Ansprüche, wobei das Gefäß durch Angioplastie, Platzieren eines Stents, Platzieren eines Shunts, oder Transplantieren eines natürlichen Transplantats, oder jeder Kombination davon, traumatisiert ist.

13. Dosierungsform nach Anspruch 11, wobei die Traumabelastung von einer Überdehnung des Gefäßes durch einen Ballonkatheter währen der Angioplastie verursacht ist.

14. Dosierungsform nach einem der vorstehenden Ansprüche, wobei die Verabreichung lokal ist.

15. Dosierungsform nach einem der vorstehenden Ansprüche, wobei das Gefäß durch einen Eingriff traumatisiert ist.

16. Kristallines Taxol mit einer Kristallgröße von 0,1 µm bis 1 mm zur Verwendung bei der Inhibierung oder Verringerung der Abnahme der Gefäßvolumenfläche bei einem durch einen Eingriff traumatisierten Säugetierblutgefäß.

17. Kristallines Taxol mit einer Kristallgröße von 0,1 µm bis 1 mm zur Verwendung bei der Inhibierung oder Verringerung der Abnahme der Gefäßvolumenfläche eines Säugetierblutgefäßes in einem durch einen Eingriff traumatisierten Säugetierblutgefäß, wobei das Taxol in *situ* mittels einer implantierbaren Vorrichtung verabreicht wird, wobei das Taxol in wiederfreisetzbarer Form in die implantierbare Vorrichtung eingebettet ist, darauf aufgeschichtet ist, oder darin eingebettet und darauf aufgeschichtet ist.

## Revendications

1. Forme pharmaceutique comprenant un taxol pour utilisation dans l'inhibition ou la réduction de la diminution de l'aire de lumière de vaisseau d'un vaisseau sanguin de mammifère, dans laquelle le taxol est sous forme sensiblement cristalline et dans laquelle la taille de cristal du taxol est de 0,1 micron à 1 mm.

2. Forme pharmaceutique de la revendication 1 dans laquelle la taille de cristal est de 1 micron à 25 microns.

3. Forme pharmaceutique des revendications 1 ou 2 dans laquelle le taxol est administré à l'aide d'un dispositif implantable.

4. Forme pharmaceutique de la revendication 3 dans laquelle le taxol est incorporé de façon réversible dans, appliqué sur, ou incorporé dans et appliqué sur, le dispositif implantable.

5. Forme pharmaceutique de la revendication 3 dans laquelle le taxol est incorporé de façon réversible dans des microparticules, des nanoparticules, ou un mélange de celles-ci.

6. Forme pharmaceutique de la revendication 5 dans laquelle les microparticules ou nanoparticules sont incorporées de façon réversible dans, appliquées sur, ou incorporées dans et appliquées sur, le dispositif implantable.

7. Forme pharmaceutique d'une des revendications 3 à 6 dans laquelle le dispositif est une enveloppe adventice, une greffe artificielle, un cathéter, une endoprothèse ou une dérivation.

8. Forme pharmaceutique d'une des revendications 3 à 6 dans laquelle le dispositif implantable est un cathéter qui est implanté pendant une durée limitée.

9. Forme pharmaceutique des revendications 1 ou 2 dans laquelle le taxol est incorporé de façon reversible dans, appliqué sur, ou incorporé dans et appliqué sur, une matrice non liquide.

10. Forme pharmaceutique d'une des revendications précédentes dans laquelle l'administration se fait avant, pendant ou après un traumatisme vasculaire procédural, ou une combinaison quelconque de ceux-ci.

11. Forme pharmaceutique d'une des revendications précédentes dans laquelle la forme pharmaceutique est administrée localement par un cathéter qui est introduit dans le vaisseau affecté pendant la procédure.

12. Forme pharmaceutique d'une des revendications précédentes dans laquelle le vaisseau est traumatisé par angioplastie, placement d'une endoprothèse, placement d'une dérivation, ou greffe naturelle, ou une combinaison quelconque de ceux-ci.

13. Forme pharmaceutique de la revendication 11 dans laquelle le choc traumatique est consécutif à un surétirage vasculaire par un cathéter à ballonnet pendant une angioplastie.

14. Forme pharmaceutique d'une des revendications précédentes dans laquelle l'administration est locale.

15. Forme pharmaceutique d'une des revendications précédentes dans laquelle le vaisseau est traumatisé de façon procédurale.

16. Taxol cristallin ayant une taille de cristal de 0,1 micron à 1 mm pour utilisation dans l'inhibition ou la réduction de la diminution du volume de lumière vasculaire dans un vaisseau sanguin de mammifère traumatisé de façon procédurale.

17. Taxol cristallin ayant une taille de cristal de 0,1 micron à 1 mm pour utilisation dans l'inhibition ou la réduction de la diminution de volume de lumière vasculaire dans un vaisseau sanguin de mammifère traumatisé de façon procédurale, le taxol étant administré *in situ* au moyen d'un dispositif implantable et le taxol étant incorporé de façon réversible dans, appliqué sur ou incorporé dans et appliqué sur le dispositif implantable.
